Europäisches Patentamt

European Patent Office

Office européen des brevets

(19)

(11) Publication number: 0 283 513 B1

## (12) EUROPEAN PATENT SPECIFICATION

(45) Date of publication of patent specification: 28.04.93

(21) Application number: 87907043.1

(22) Date of filing: 16.09.87

(86) International application number:
PCT/US87/02347

(87) International publication number:
WO 88/01861 (24.03.88 88/07)

(51) Int. Cl.$^5$: **A61K 9/10**, A61K 31/195,
A61K 31/20, A61K 31/70,
A61K 37/02

(54) NUTRITIONAL SUPPORT OR THERAPY FOR INDIVIDUALS AT RISK OR UNDER TREATMENT FOR ATHEROSCLEROTIC, VASCULAR, CARDIOVASCULAR, AND/OR THROMBOTIC DISEASES.

(30) Priority: 17.09.86 US 908447

(43) Date of publication of application:
28.09.88 Bulletin 88/39

(45) Publication of the grant of the patent:
28.04.93 Bulletin 93/17

(84) Designated Contracting States:
AT BE CH DE FR GB IT LI

(56) References cited:
EP-A- 0 189 160
US-A- 4 434 160
US-A- 4 526 902
US-A- 4 687 782
US-A-44 381 44

Dictionnaire Vidal 1982, p. 5

(73) Proprietor: CLINTEC NUTRITION COMPANY
Three Parkway North, Suite 500
Deerfield, Illinois 60015-0760(US)

(72) Inventor: COTTER, Richard
1717 Cedar Glen Drive
Libertyville, IL 60048(US)
Inventor: JOHNSON, Robert, C.
1107 Hull Avenue
Westchester, IL 60153(US)
Inventor: WARD, Michael
427 West Stratford Court
McHenry, IL 60050(US)
Inventor: MADSEN, David, C.
600 Ardmore Terrace
Libertyville, IL 60048(US)
Inventor: VALICENTI, Anthony, J.
400 Margate Terrace
Deerfield, IL 60015(US)
Inventor: MENARD, Michael, P.
58 George Street
Grayslake, IL 60030(US)

Inventor: **TUCKER, Hugh, N.**
**25950 West Hippler**
**Barrington, IL 60010(US)**

(74) Representative: **Bassett, Richard Simon**
**ERIC POTTER & CLARKSON St. Mary's Court**
**St. Mary's Gate**
**Nottingham NG1 1LE (GB)**

## Description

The present invention relates to nutritional formulations for the support and therapy of individuals. More specifically, the present invention relates to nutritional compositions for supporting and/or providing therapy to individuals at risk and/or under treatment for atherosclerotic, vascular, cardiovascular, or thrombotic diseases.

For some time investigators and scientists have noted a relationship between diet and the heart function and related systems. US4526902 relates to pharmaceutical compositions or food products containing a specific combination of fatty acids which can be used to treat or provide effective prophylaxis against thromboembolic conditions. There has always been an appreciation for the cardiovascular effects of obesity and the recognition of widespread prevalence of undernutrition in hospitalized patients with cardiovascular derangements. Accordingly, there have been many attempts to formulate nutritional support for patients at risk for or exhibiting atherosclerotic, vascular, cardiovascular, and/or thrombotic diseases. Poindexter, et al, Nutrition in Congestive Heart Failure, Nutrition In Clinical Practice (1986) recognize that specific nutritional deficiencies may cause, precipitate, or aggravate acute heart failure. As Poindexter, et al, point out, nutritional deficiencies have been significant factors in the etiology of heart failure in the Orient and developing countries. It is further noted that nutritional therapy for malnourished cardiac patients in recent years has been considered essential supportive therapy.

Patients suffering from long term congestive heart failure have been found to suffer from cardiac cachexia. Other effects of protein-calorie malnutrition on the heart include hypertension, reduced heart rate, reduction in basal metabolic rate and oxygen consumption, atrophy of the heart muscle mass, electrocardiogram (ECG) abnormalities, and heart failure. Furthermore, when congestive heart failure occurs secondary to valvular heart disease that is treated surgically, nutritional status has a notable effect on the surgical outcome. Performing cardiac surgical procedures on patients in a state of nutritional depletion can result in increased morbidity and mortality, compared to adequately nourished patients.

Typically, patients suffering from congestive heart failure are underweight with poor nutritional status. Patients with congestive heart failure and cardiac cachexia frequently exhibit anorexia and early satiety. Poindexter, et al, state that this is attributed to the natural compensatory mechanism that decreases work of the failing heart. Furthermore, due to hepatic congestion that increases pressure in the abdominal cavity, there is a constant feeling of fullness. Moreover, altered taste sensations and intolerances to food odors limit the patient's desire to eat. Accordingly, liquid nutritional supplements high in nutrient density are desirable. However, as Poindexter notes, this must be tempered with concern about the complications caused by overzealous refeeding of malnourished cardiac patient.

Not only are patients with congestive heart failure and other vascular diseases typically underweight with poor nutritional status, but their energy requirements are greatly in excess of a normal individual's energy requirements. Poindexter, et al, note that energy requirements of a patient with congestive heart failure may be 30-50 percent in excess of basal energy expenditure because of increased cardiac and pulmonary energy expenditure. Indeed, cachectic patients require additional calories for repletion and post-operative cardiac patients require still further increases in caloric intake to meet energy demands. For example, the protein requirement for a normal healthy individual to maintain zero nitrogen balance is 0.5-1.0g/Kg. The patient with congestive heart failure or the post-operative cardiac patient in contrast can require as much as 1.5-2.0g/Kg to maintain nitrogen balance.

Not only is nutrition important in treating the patient with atherosclerotic, vascular, cardiovascular, and/or thrombotic diseased but it is also important in supporting patients at risk of acquiring these diseases. Diet can impact the onset of these diseases in certain individuals.

EP0189160 describes an improved high fat, low carbohydrate enteral nutritional composition which is utilized in the dietary management of medical patients requiring a reduced carbohydrate intake. There is now a need for a nutritional composition for supporting and therapeutically treating individuals under treatment for vascular, cardiovascular, or thrombotic diseases. Moreover, there is a need for a nutritional composition for supporting individuals who are at high risk of atherosclerotic, vascular, cardiovascular, and/or thrombotic disease.

The features of the present invention are defined in the characterising part of Claim 1, in which the precharacterising part is based on EP-A-0189160, and are also defined in Claims 15, 22 and 23.

The present invention provides a nutritional composition that affords a rational, scientific diet or supplement for individuals at high risk or under treatment for atherosclerotic, vascular, cardiovascular, and/or thrombotic diseases. The formulation is designed to slow the progression of these diseases, and prevent the onset of acute episodes that can result in the death of such patients. To this end, the present invention provides a composition that includes nutritional subunits that have been shown to effect various

3

biochemical and physiological parameters of vascular, cardiovascular and blood systems. The formulation can be administered either as an enteral product or parenterally.

As an enteral product, the formulation comprises a protein source comprising a solution of amino acids, a carbohydrate source, a fat source, and preferably electrolytes. The protein source preferably includes a high biological value protein, an amino acid solution, branched-chain amino acids, and carnitine. The high biological value protein comprises the base component. Although any high biological value protein can be utilized preferably the high biological value protein is lactalbumin or soy protein. Whole protein or hydrolysates can be utilized.

The amino acid solution is designed to provide the essential, conditionally essential, and non-essential amino acids necessary for efficacious protein metabolism in the face of cardiovascular or thrombotic disease states. The amino acid solution preferably includes: L-Arginine; L-Leucine; L-Isoleucine; L-Lysine; L-Valine; L-Phenylalanine; L-Histidine; L-Threonine; L-Methionine; L-Tryptophan; L-Alanine; L-Proline; L-Serine; L-Tyrosine; and amino acetic acid. An example of an amino acid solution formulation that will function satisfactorily is TRAVASOL$^R$ marketed by Travenol Laboratories, Deerfield, Illinois. Of course, depending upon requirements not all of the amino acids must be included in the solution. Of course, other nutrients such as, for example, biologically available sources of taurine and cysteine can be added. Preferably the arginine:lysine ratio is between approximately about 0.7:1 to 1.25:1. Most preferably the ratio is approximately 1:1. Clinical and experimental evidence has shown that an arginine:lysine ratio of 1 to 1 is associated with lower plasma cholosterol levels.

The amino acid and base protein, i.e., high biological value protein, is combined with branched-chain amino acids to achieve a final concentration of approximately 45 to 55 percent branched-chain amino acids (w/w). Most preferably the final concentration of branched-chain amino acids is 50 percent of total protein and amino acid content. The branched-chain amino acid mixture that function satisfactorily is that capable of maintaining essential intake of all three branched-chain amino acids to meet nutritional requirements. The branched-chain amino acids Isoleucine, Leucine, and Valine are preferably included in a 1:1:1 molar ratio. An example of such a branched-chain amino acid formula is BRANCHAMIN$^R$ marketed by Travenol Laboratories, Deerfield, Illinois. Observations on rats and dogs demonstrate that these cardiac muscles depend more on branched-chain amino acids than on all other amino acids. As previously stated, other amino acids can be utilized; for example, in neonates and infants it may be desirable to include taurine.

Preferably glycine should be supplemented to the protein source, if necessary, to obtain levels typically found in soy protein. It has been found that higher levels of plasma glycine are associated with lowered levels of plasma cholesterol.

The protein source also preferably includes L-carnitine. The L-carnitine is added to achieve a final concentration of approximately 15 to 40 mg/g of total protein. Most preferably the final concentration of L-carnitine is 25 mg/g of total protein. Many publications have shown that damaged cardiac muscle functions better when supplemented with L-carnitine.

The nutritional composition also contains a carbohydrate source. The carbohydrate source preferably includes xylitol and a glucose-based carbohydrate. In a preferred embodiment the carbohydrate source includes maltodextrin and xylitol. The glucose substrate and xylitol are preferably present in a 1:1 ratio by weight. The carbohydrate source can also include ribose. In a preferred embodiment, the composition contains maltrodextrin, xylitol, and ribose in a prefered ratio of approximately 1:1:.066 by weight. In another embodiment, the composition contains maltrodextrin and xylitol preferably in a ratio of approximately 1:1 by weight

The use of carbohydrates such as xylitol or ribose in nutritional support of individuals susceptible to and/or under treatment for cardiovascular disease is based upon the unique pathways for the metabolism of these compounds. Xylitol is a naturally occurring intermediate in the glucuronic acid-xylulose cycle, and may also be metabolized through the generation of the intermediate compound xylulose to form ribose. Accordingly, the administration or ingestion of the xylitol, xylulose, and/or ribose provides conversions of these intermediates to glucose. By providing a glucose-based carbohydrate source, i.e., maltodextrin, conversion of these compounds to glucose is minimized. The administration of a 1 to 1 ratio of glucose subunits with xylitol and ribose represents an effective means to maximize glucose production with minimal insulin elevation, while enhancing adenine nucleotide synthesis for this patient population.

The effect of ribose on cardiac function and ischemic events may be related to several specific functions of the compound. Administration of ribose to cardiac tissue following oxygen deprivation has been demonstrated to result in a 90% increase in the de novo synthesis of myocardial adenine nucleotides, as well as in the elevation of 5-phosphoribosyl-1-pyrophosphate (PRPP) specific pool in myocardial tissue. Continuous infusion of ribose has been demonstrated to result in a 13-fold increase in myocardial adenine nucleotide synthesis. Such elevations have further been demonstrated to reduce the occurrence of cell lesions in the myocardium.

It has been suggested that cellular depletion of compartmentalized ATP may be primarily responsible for the pathological effects of the ischemic event, through an imbalance between subcellular phosphocreatine and compartmentalized ATP. ATP may also serve as a modulator of myocardial cell function, responsible for potassium exchange and calcium:sodium exchange. These reactions require higher concentrations of ATP than those required for the PRPP pool alone. Demonstration of a marked effect of ribose administration on protection against isoproterenol-induced myocardial cell damage further supports the hypothesis for a role in cellular depletion of adenine nucleotides in the progression of cardiac necrosis.

The lipid component of the nutritional composition comprises long chain triglycerides and medium chain fatty acids. Preferably, the long chain triglycerides comprise "marine oils" and/or gamma-linolenic acid of 11 to 26 carbons in length. These fatty acids can be both saturated and unsaturated in nature. It has been shown that monounsaturated fatty acids are effective in lowering plasma cholesterol. Accordingly, preferably monounsaturated fatty acids are utilized as a component of these lipid substrates.

The medium chain fatty acids preferable in the present invention are those that are 6 to 10 carbons in length. These medium chain fatty acids are a superior energy source for the cardiac muscle cells. The fatty acids can be provided to patients as free fatty acids, mono-, di- or triglycerides. Medium chain fatty acids are chemically unique in that in the absence of cytoplasmic medium chain fatty acyl CoA synthetase they are able to pass through the inner mitochondrial membrane unhindered. Medium chain fatty acyl CoA synthetase does exist in the mitochondria and activates the fatty acids once they have crossed the inner membrane. These activated fatty acids are then rapidly metabolized.

In contrast, long chain fatty acids, i.e., those fatty acids having 11 to 26 carbons, due to their chemical nature cannot cross the inner mitochondrial membrane without being first activated by cytoplasmic long chain fatty acyl CoA synthetase, a rate limiting process. The long chain fatty acids must then undergo obligatory conversion to a carnitine transport form for entry into the mitochondria for metabolism.

Medium chain fatty acids combine their unique ability to cross the mitochondria membrane with the unique biochemical milieu of the cardiac cell. The cardiac muscle lacks cytoplasmic medium chain acyl CoA synhetase and the ability to activate medium chain fatty acids. Thus, medium chain fatty acids rapidly enter the mitochondria and supply energy in these cells directly. Long chain fatty acids cannot do this because of their necessary cytoplasmic activation and the slower carnitine transport in this organ.

The long chain triglycerides comprise marine oils and/or gamma-linolenic acid (GLA) and/or sterodonic acid. The marine oils preferably include linolenic acid and large amounts of two other members of the omega three family: eicosapentaenoic acid (EPA) and docosahexaenoic acid (DHA). These fatty acids are incorporated into cell membranes and serum lipids and give rise to metabolites of the omega-three metabolic pathways. GLA is an omega-6 fatty acid and is a precursor to the 1-series prostaglandins.

The long chain triglycerides comprise from 50% to 25% of the lipid component and the medium chain fatty acid comprises preferably from 75% to 50% of the lipid component. If GLA is utilized with marine oil, preferably approximately three times as much marine oil is used as GLA.

All cells utilize these fatty acids to form various prostaglandins and leukotrienes. When fatty acids are released from cell membranes, lipoxygenase and cyclooxygenase mediate further metabolic activity. Although EPA is a relatively poor substrate for lipoxygenase and cyclooxygenase, it appears to have a high binding affinity and thereby inhibits arachidonic acid conversion by these enzymes. An added benefit of the omega three fatty acid pathway lies in the physiological activity of their cellular products (See Table I - $PGI_2$ = 2-series prostacyclin; $PGI_3$ = 3-series prostacyclin; $TXA_2$ = 2-series thromboxane; and $TXA_3$ = 3-series thromboxane).

TABLE I

| Cell | Fatty Acid | Product | Physiological Actions |
|------|-----------|---------|----------------------|
| Endothelial | Arachidonic | $PGI_2$ | Lower platelet activity: vasodilation |
|  | Eicosapentaenoic | $PGI_3$ | Lower platelet activity: vasodilation |
| Platelet | Arachidonic | $TXA_2$ | platelet hyperactivity: vasoconstriction |
|  | Eicosapentaenoic | $TXA_3$ | Lower platelet activity: vasoconstriction |

In most subjects who consume such diets, total serum cholesterol, LDL cholesterol, and triglycerides are significantly lowered, whereas HDL cholesterol concentrations are elevated. This pattern of change would be one thought to be less atherogenic and the thrombogenic.

Studies conducted with human platelets utilizing pure EPA and arachidonic acid support the role of the balance of EPA and arachidonic acid as the critical factor in controlling platelet activators and vessel constriction.

The electrolytes component of the present invention preferably includes sodium, potassium, chloride, calcium, magnesium, and phosphorus.

Preferably the protein source comprises approximately 15 to about 25% of the caloric source of the enteral nutritional composition. Most preferably the protein source comprises approximately 20% of the caloric source of the enteral nutritional composition. Preferably, the carbohydrate source comprises approximately 40% to about 75% of the caloric source of the enteral nutritional composition. Most preferably, the carbohydrate source comprises aproximately 50% of the caloric source of the enteral nutritional composition. Preferably the lipid component comprises approximately 10% to about 40% of the caloric source of the enteral nutritional composition. Most preferably the lipid component comprises approximately 30% of the caloric source of the enteral nutritional composition.

By way of example two preferred enteral cardiac formulations will now be set forth.

## TABLE II

### CARDIAC FORMULATION

| | |
|---|---|
| **Form:** | Liquid |
| **Concentration:** | 2.0 kcal/ml |
| **Protein Source:** | Lactalbumin |
| | L-Carnitine |
| | Enhance BCAA |
| | Hi Arg: lys ratio |
| | Inc. Glycine |
| Gm/Liter | 100 |
| % Cal | 20 |
| **Carbohydrate Source:** | Maltodextrin |
| | Xylitol, |
| | Ribose |
| Ratio: | 1.0:1.0:.066 |
| Gm/Liter | 121,121,8 |
| % Cal | 50 |
| **Fat Source:** | Marine Oil (MO) |
| | GLA, MCT |
| Gm/Liter MCT&LCT | 57.7 |
| MO:GLA:MCT | 3:1:12 |
| % Cal | 30 |

Electrolytes:

| | | |
|---|---|---|
| Na/Liter | 500mg | |
| | 21.8mEq | |
| K/Liter | 1000mg | |
| | 35.4mEq | |
| Cl/Liter | 1000mg | |
| Ca/Liter | 1200mg | |
| P/Liter | 1000mg | |
| Mg/Liter | 600mg | |

### TABLE III

CARDIAC FORMULATION

| Form: | Liquid |
|---|---|

| Concentration: | 2.0 kcal/ml |
|---|---|

| Protein Source: | Lactalbumin |
|---|---|
| | L-Carnitine |
| | Enhance BCAA |
| | Hi Arg: lys ratio |
| | Inc. Glycine |
| Gm/Liter | 100 |
| % Cal | 20 |

| Carbohydrate Source: | Maltodextrin |
|---|---|
| | Xylitol |
| Ratio: | 1.0:1.0 |
| Gm/Liter | 125,125 |
| % Cal | 50 |

| Fat Source: | Marine Oil (MO) |
|---|---|
| | GLA, MCT |

| | |
|---|---|
| Gm/Liter MCT&LCT | 57.7 |
| MO:GLA:MCT | 3:1:12 |
| % Cal | 30 |

| Electrolytes: | |
|---|---|
| Na/Liter | 500mg |
| | 21.8mEq |
| K/Liter | 1000mg |
| | 35.4mEq |
| Cl/Liter | 1000mg |
| | 28.3mEq |
| Ca/Liter | 1200mg |
| P/Liter | 1000mg |
| Mg/Liter | 600mg |

The parenteral regimen for the composition for providing nutritional support or therapy for individuals at risk or under therapy for vascular, cardiovascular, or thrombotic disease is preferably modular. However, the parenteral regimen can be premixed before use. The parenteral regimen solution for injection contains: a lipid emulsion; a carbohydrate solution; carnitine; branched-chain amino acids; and amino acids.

Preferably, the lipid emulsion for injection includes approximately 5 to about 20% of a triacylglycerol oil containing approximately 5 to about 80% eicosapentaenoic acid (EPA) and/or approximately 5 to about 80% gamma-linolenic acid (GLA) and approximately 3 to about 25% sterodonic acid (6, 9, 12, 15-octadecatetraenoic acid), with approximately 0.4 to about 1.6% egg or soy bean phospholipid and approximately 2.25% of glycerol or other physiologically acceptable tonicity agent, adjusted to physiological pH with sodium hydroxide. Water or water and medium chain triglycerides comprise the remainder of the lipid emulsion. If medium chain triglycerides are used they comprise no more than 30% (w/v) of the lipid emulsion.

The carbohydrate injection solution preferably contains glucose and xylitol in an approximately 1:1 ratio by weight. The solution can contain in an embodiment approximately 3.3% (w/v) ribose.

To the branched-chain amino acid injection solution can be added any other amino acid capable of necessary to meet nutritional requirements. The branched-chain amino acid injection solution contains Isoleucine, Leucine, and Valine, preferably in a 1:1:1 molar ratio.

The solution for injection of amino acids can contain essential, non-essential, and conditionally essential amino acids. Preferably the solution includes: L-Arginine; L-Leucine; L-Isoleucine; L-Lysine; L-Valine; L-Phenylalanine; L-Histidine; L-Threonine; L-Methionine; L-Tryptophan; L-Alanine; L-Proline; L-Serine; L-Tyrosine; and amino acetic acid. However, the solution can contain less than all these amino acids, or other nutrients such as, for example, taurine and cysteine An example of such an amino acid solution and the relevant proportions of each amino acids is TRAVASOL[R] marketed by Travenol Laboratories, Deerfield, Illinois.

By way of example contemplated examples will now be given.

Example One

This contemplated example demonstrates the use of the parenteral cardiac formulation in providing nutrition and therapy to a patient suffering cardiovascular disease.

A middle-aged male patient is admitted to intensive care following an acute myocardial infarction. Among the therapies administered would be the parenteral cadiac formulation as part of a continuous intravenous infusion. The parenteral cardiac formula includes: a lipid emulsion injection; a carbohydrate injectable solution; injectable carnitine; injectable branched-chain amino acid solution; and an injectable amino acid solution. The lipid emulsion for injection includes 10% of a triacylglycerol oil containing 15% eicosapentaenoic acid (EPA) and 5% gamma-linolenic acid (GLA) and 5% sterodonic acid with 1.2% soybean phospholipid and approximately 2.25% of glycerol and water. The carbohydrate injection solution contains glucose and xylitol in an approximately 1:1 ratio by weight. The branched-chain amino acid injection solution contains Isoleucine, Leucine, and Valine, in a 1:1:1 molar ratio. The amino acid solution was TRAVASOL[R]. The key critical features of this patient's clinical profile include:

cardiac ischemia with hyperreactive platelets that can be easily triggered to aggregate, leading to a life-threatening secondary event involving thrombus formation and vasoconstriction of the coronary artery at the site of activation, increased vascular tone and a predisposition to vascular spasm.

As a result of this cardiac parenteral formulation, the patient's cardiac muscle tissue would have available energy and protein substrates and their platelets would be far less reactive within hours of the onset of I.V. administration. Furthermore, the balance of the 2-series prostacyclins and 3-series prostacyclin/2-series thromboxane ratio would begin to shift in a favorable direction, leading to a reduced risk of vascular spasm.

## Example Two

This same patient, as described in example one, recovers and is sent home to follow a strict regimen. He has advanced atherosclerosis, the sequellae of which include hypertension, elevated serum triglycerides and LDL, VLDL, and total cholesterol concentrations, low serum HDL cholesterol concentration, and a very high risk of stroke, myocardial infarction, or other thrombotic events.

Doctors focus on dietary control of this disease process, to supplement prescribed medications. The cardiac enteral diet set forth in Table II as a nutritional supplement provides necessary cardiac muscle nutrition as well as the therapeutic effects of EPA/DHA. Consumed on a daily basis, this diet would:

1. provide specialized cardiac muscle protein;
2. provide carbohydrate and calorie nutrition;
3. lower serum triglyceride and LDL, VLDL, and total cholesterol concentrations;
4. markedly reduce platelet reactivity, leading to reduced incidence of thromboxane and serotonin release by platelets (vasoactive stimulators and platelet activators) as well as platelet derived growth factor release (a known atherogenic factor);
5. lower systolic blood pressure, another factor associated with atherogenesis.

## Example Three

In this contemplated example, a patient with cardiovascular disease requires a vascular graft. The highest risk for graft-associated thrombosis occurs within the first week following graft placement. Since this is a platelet/white blood cell-mediated event, lacing this patient on a combined parenteral (set forth in Example One) and enteral (Table II) cardiac formulation for 7-10 days, while in recovery, will markedly dampen both platelet and white blood cell reactivity as well as provide essential nutritional support.

Following release from hospital, this patient could continue with the daily consumption of the parenteral and/or enteral formulation to maintain a low thrombogenic potential.

## Example Four

In this contemplated example, an elderly patient following hip surgery is committed to several weeks of bed rest. There is a recognized marked thrombotic tendency following this procedure, partly due to the surgery itself, and partly to the prolonged vascular stasis resulting from the elimination of physical activity.

A regimen of combined enteral (Table II) and parenteral (set forth in Example One) cardiac formulation for a week following surgery, and a continuation of the enteral formulation during the remainder of the recovery period, will not only dampen the thrombotic tendency, but also will provide essential nutrients to support the healing process in this elderly patient.

## Claims

1. An enteral nutritional composition comprising therapeutically effective amounts of a protein source, a carbohydrate source and a lipid component including at least one medium chain fatty acid, characterised in that the composition is formulated for individuals under treatment for, or at risk of, atherosclerotic, vascular, cardiovascular, and/or thrombotic disease and the lipid component comprises long chain triglycerides comprising gamma-linolenic acid and/or sterodonic acid and/or marine oil in an amount comprising 50% to 25% of the lipid component.

9

2. An enteral nutritional composition according to Claim 1, wherein
the protein source represents 15 to 25% of the caloric source of the composition; the protein source including essential, conditionally essential, and nonessential amino acids, branched-chain amino acids, and a high biological value protein;
the carbohydrate source represents 40% to 75% of the caloric source of the composition; and
the lipid component represents 10% to 40% of the caloric source of the composition, the lipid component including medium chain fatty acids.

3. The nutritional composition of Claim 1 or 2 wherein the marine oil contains at least one of eicosapentaenoic acid, docosahexaenoic acid and linolenic acid.

4. The nutritional composition of any one of the preceding claims wherein the protein source includes;
a high biological value protein;
amino acids;
branched-chain amino acids; and
L-carnitine.

5. The nutritional composition of Claim 4 wherein the high biological value protein is lactalbumin or soy protein.

6. The nutritional composition of Claim 4 or 5 wherein the amino acids include: L-arginine; L-leucine; L-isoleucine; L-lysine, L-valine; L-phenylalanine; L-histidine; L-threonine; L-methionine; L-tryptophan; L-alanine; L-proline; L-serine; L-tyrosine; and amino acetic acid.

7. The nutritional composition of Claim 4 or 5 wherein the branched-chain amino acids include: isoleucine, leucine, and valine.

8. The nutritional composition of any one of the preceding claims wherein the carbohydrate source includes glucose subunits and xylitol.

9. The nutritional composition of Claim 8 wherein the glucose subunits is maltodextrin.

10. The nutritional composition of any one of the preceding claims wherein the carbohydrate source includes ribose.

11. The nutritional composition of any one of the preceding claims wherein the medium chain fatty acid comprises 50 to 75% of the total lipid content of the composition.

12. The nutritional composition of any one of the preceding claims including a therapeutically effective amount of electrolytes.

13. The composition of any one of the preceding claims being an enteral composition wherein the protein source includes L-carnitine.

14. The composition of Claim 3 or any claim when dependent thereon, being an enteral composition and further including gamma-linolenic acid and sterodonic acid.

15. A parenteral regimen for cardiac therapy comprising therapeutically effective amounts of:
an injectable lipid emulsion including a triacylglycerol oil having at least one of the lipids selected from the group consisting of eicosapentaenoic acid, gamma-linolenic acid and sterodonic acid, and a phospholipid chosen from the group consisting of egg phospholipid or soybean phospholipid, and glycerol and water, the triacylglycerol oil comprising 5 to 20% of the lipid emulsion;
an injectable solution of a carbohydrate;
L-carnitine;
an injectable solution of branched-chain amino acids; and
an injectable solution of amino acids.

10

**16.** The parenteral regimen of Claim 15 wherein the triacylglycerol oil includes 5 to 80% eicosapentaenoic acid, and/or 5 to 80% gamma-linolenic acid and/or 3 to 35% sterodonic acid.

**17.** The parenteral regimen of Claim 15 or 16 wherein the amino acids include: L-arginine; L-leucine; L-isoleucine; L-lysine; L-valine; L-phenylalanine; L-histidine; L-threonine; L-methionine; L-tryptophan; L-alanine; L-proline; L-serine; L-tyrosine; and amino acetic acid.

**18.** The parenteral regimen of Claim 15 to 16 wherein the branched-chain amino acids include: isoleucine, leucine, and valine.

**19.** The parenteral regimen of any one of Claims 15 to 18 wherein the lipid emulsion, carbohydrate solution, L-carnitine, branched-chain amino acids and amino acids are premixed before infusion into a patient.

**20.** The parenteral regimen of any one of Claims 15 to 19 wherein the lipid emulsion includes medium chain triglycerides.

**21.** The parenteral regimen of any one of Claims 15 to 20, where the carbohydrate solution comprises glucose and xylitol.

**22.** Use in the manufacture of an enteral nutritional composition of therapeutically effective amounts of a protein source, a carbohydrate source and a lipid component including at least one medium chain fatty acid and long chain triglycerides comprising gamma-linolenic acid and/or sterodonic acid and/or marine oil in an amount comprising 50% to 25% of the lipid component, for the purpose of nutrition of individuals under treatment for, or at risk of, atherosclerotic, vascular, cardiovascular, and/or thrombotic disease.

**23.** Use in the manufacture of a parenteral regimen of therapeutically effective amounts of a lipid emulsion including a triacylglycerol oil having at least one of the lipids selected from eicosapentaenoic acid, gamma-linolenic acid and sterodonic acid, and a phospholipid chosen from egg phospholipid or soybean phospholipid, and glycerol and water, the triacylglycerol oil comprising 5 to 20% of the lipid emulsion ; an injectable solution of a carbohydrate ; L-carnitine ; an injectable solution of branched-chain amino acids ; and an injectable solution of amino acids for the purpose of nutrition of individuals under treatment for, or at risk of, atherosclerotic, vascular, cardiovascular, and/or thrombotic disease.

**Patentansprüche**

**1.** Enterale Ernährungszusammensetzung, die therapeutisch wirksame Mengen einer Proteinquelle, einer Kohlenhydratquelle und einer Lipidkomponente mit wenigstens einer mittelkettigen Fettsäure aufweist, dadurch gekennzeichnet,
daß die Zusammensetzung für Einzelpersonen formuliert ist, die wegen atherosklerotischer, Gefäß-, Kreislauf- und/oder thrombotischer Krankheit behandelt werden oder ein solches Krankheitsrisiko haben, und daß die Lipidkomponente langkettige Triglyceride aufweist, die Gamma-Linolensäure und/oder Sterodonsäure und/oder Seetieröl in einer Menge aufweisen, die 50 % bis 25 % der Lipidkomponente aufweist.

**2.** Enterale Ernährungszusammensetzung nach Anspruch 1, wobei:
- die Proteinquelle 15 bis 25 % der kalorischen Quelle der Zusammensetzung repräsentiert; wobei die Proteinquelle essentielle, bedingt essentielle und nichtessentielle Aminosäuren, verzweigtkettige Aminosäuren und ein biologisch hochwertiges Protein aufweist;
- die Kohlenhydratquelle 40 bis 75 % der kalorischen Quelle der Zusammensetzung repräsentiert; und
- die Lipidkomponente 10 bis 40 % der kalorischen Quelle der Zusammensetzung repräsentiert, wobei die Lipidkomponente mittelkettige Fettsäuren aufweist.

**3.** Ernährungszusammensetzung nach Anspruch 1 oder 2, wobei das Seetieröl wenigstens eine der folgenden Säuren aufweist: Eicosapentaensäure, Docosahexaensäure und Linolensäure.

4. Ernährungszusammensetzung nach einem der vorhergehenden Ansprüche, wobei die Proteinquelle folgendes aufweist:
   - ein biologisch hochwertiges Protein;
   - Aminosäuren;
   - verzweigtkettige Aminosäuren; und
   - L-Carnitin.

5. Ernährungszusammensetzung nach Anspruch 4, wobei das biologisch hochwertige Protein Lactalbumin oder Sojaprotein ist.

6. Ernährungszusammensetzung nach Anspruch 4 oder 5, wobei die Aminosäuren die folgenden umfassen: L-Arginin; L-Leuzin; L-Isoleuzin; L-Lysin; L-Valin; L-Phenylalanin; L-Histidin; L-Theronin; L-Methionin; L-Tryptophan; L-Alanin; L-Prolin; L-Serin; L-Tyrosin; und Aminoessigsäure.

7. Ernährungszusammensetzung nach Anspruch 4 oder 5, wobei die verzweigtkettigen Aminosäuren die folgenden aufweisen: Isoleuzin, Leuzin und Valin.

8. Ernährungszusammensetzung nach einem der vorhergehenden Ansprüche, wobei die Kohlenhydratquelle Glukose-Untereinheiten und Xylitol aufweist.

9. Ernährungszusammensetzung nach Anspruch 8, wobei die Glukosequelle Maltodextrin ist.

10. Ernährungszusammensetzung nach einem der vorhergehenden Ansprüche, wobei die Kohlenhydratquelle Ribose aufweist.

11. Ernährungszusammensetzung nach einem der vorhergehenden Ansprüche, wobei die mittelkettige Fettsäure 50 bis 75 % des gesamten Lipidgehalts der Zusammensetzung aufweist.

12. Ernährungszusammensetzung nach einem der vorhergehenden Ansprüche, die eine therapeutisch wirksame Menge von Elektrolyten aufweist.

13. Zusammensetzung nach einem der vorhergehenden Ansprüche, die eine enterale Zusammensetzung ist, wobei die Proteinquelle L-Carnitin aufweist.

14. Zusammensetzung nach Anspruch 3 oder einem darauf rückbezogenen Anspruch, die eine enterale Zusammensetzung ist und außerdem Gamma-Linolensäure und Sterodonsäure aufweist.

15. Parenterales Therapieschema für die Herztherapie, das therapeutisch wirksame Mengen von folgenden Substanzen aufweist:
   - einer injizierbaren Lipidemulsion, die folgendes aufweist: ein Triacylglycerinöl mit wenigstens einem der Lipide, die ausgewählt sind aus der Gruppe, die aus Eicosapentaensäure, Gamma-Linolensäure und Sterodonsäure besteht, und ein Phospholipid, das ausgewählt ist aus der Gruppe, die aus Ei-Phospholipid oder Sojabohnen-Phospholipid besteht, und Glycerin und Wasser, wobei das Triacylglycerinöl 5 bis 20 % der Lipidemulsion aufweist;
   - einer injizierbaren Lösung eines Kohlenhydrats;
   - L-Carnitin;
   - einer injizierbaren Lösung von verzweigtkettigen Aminosäuren; und
   - einer injizierbaren Lösung von Aminosäuren.

16. Parenterales Therapieschema nach Anspruch 15, wobei das Triacylglycerinöl 5 bis 80 % Eicosapentaensäure und/oder 5 bis 80 % Gamma-Linolensäure und/oder 3 bis 35 % Sterodonsäure aufweist.

17. Parenterales Therapieschema nach Anspruch 15 oder 16, wobei die Aminosäuren die folgenden umfassen: L-Arginin; L-Leuzin; L-Isoleuzin; L-Lysin; L-Valin; L-Phenylalanin; L-Histidin; L-Threonin; L-Methionin; L-Tryptophan; L-Alanin; L-Prolin; L-Serin; L-Tyrosin; und Aminoessigsäure.

18. Parenterales Therapieschema nach Anspruch 15 oder 16, wobei die verzweigtkettigen Aminosäuren folgende aufweisen: Isoleuzin, Leuzin und Valin.

**19.** Parenterales Therapieschema nach einem der Ansprüche 15 bis 18, wobei die Lipidemulsion, die Kohlenhydratlösung, L-Carnitin, verzweigtkettige Aminosäuren und Aminosäuren vor dem Infundieren in einen Patienten vorgemischt werden.

**20.** Parenterales Therapieschema nach einem der Ansprüche 15 bis 19, wobei die Lipidemulsion mittelkettige Triglyceride aufweist.

**21.** Parenterales Therapieschema nach einem der Ansprüche 15 bis 20, wobei die Kohlenhydratlösung Glukose und Xylitol aufweist.

**22.** Verwendung, bei der Herstellung einer enteralen Ernährungszusammensetzung, von therapeutisch wirksamen Mengen einer Proteinquelle, einer Kohlenhydratquelle und einer Lipidkomponente, die folgendes aufweist: wenigstens eine mittelkettige Fettsäure und langkettige Triglyceride, die Gamma-Linolensäure und/oder Sterodonsäure und/oder Seetieröl in einer Menge enthalten, die 50 % bis 25 % der Lipidkomponente aufweist, zum Zweck der Ernährung von Einzelpersonen, die wegen atherosklerotischer, Gefäß-, Kreislauf- und/oder thrombotischer Krankheit behandelt werden oder ein solches Krankheitsrisiko haben.

**23.** Verwendung, bei der Herstellung eines parenteralen Therapieschemas, von therpeutisch wirksamen Mengen einer Lipidemulsion, die folgendes aufweist: ein Triacylglycerinöl mit wenigstens einem der Lipide, die ausgewählt sind unter Eicosapentaensäure, Gamma-Linolensäure und Sterodonsäure, und ein Phospholipid, das ausgewählt ist unter Ei-Phospholipid oder Sojabohnen-Phospholipid, und Glycerin und Wasser, wobei das Triacylglycerinöl 5 bis 20 % der Lipidemulsion aufweist; eine injizierbare Lösung eines Kohlenhydrats; L-Carnitin; eine injizierbare Lösung von verzweigtkettigen Aminosäuren; und eine injizierbare Lösung von Aminosäuren, für die Zwecke der Ernährung von Einzelpersonen, die wegen atherosklerotischer, Gefäß-, Kreislauf- und/oder thrombotischer Krankheit behandelt werden oder ein solches Krankheitsrisiko haben.

## Revendications

**1.** Préparation nutritionnelle entérale comprenant des quantités thérapeutiquement efficaces d'une source protidique, une source d'hydrates de carbone, et un composant lipidique contenant au moins un acide gras à chaîne moyenne, caractérisée en ce que la préparation est indiquée pour les individus susceptibles de souffrir de maladies athéroscléreuses, vasculaires, cardiovasculaires et/ou thrombotiques ou en traitement pour ces maladies, et en ce que le composant lipidique comprend des triglycérides à longue chaîne contenant de l'acide gamma-linoléique et /ou de l'acide stérodonique et/ou de l'huile de poisson en quantité comprise entre 50 % et 25 % du composant lipidique.

**2.** Préparation nutritionnelle entérale selon la revendication 1 dans laquelle
   - la source protidique représente 15 à 25 % de la source calorique de la préparation; la source protidique contenant des acides aminés essentiels, conditionnellement essentiels, et non essentiels, des acides aminés à chaîne ramifiée, et une protéine de haute valeur biologique.
   - la source d'hydrates de carbone représente 40 à 75 % de la source calorique de la préparation; et le composant lipidique représente 10 à 40 % de la source calorique de la préparation, le composant lipidique contenant des acides gras à chaîne moyenne.

**3.** Préparation nutritionnelle selon les revendications 1 ou 2 dans laquelle l'huile de poisson contient au moins un acide eicosapentaenoïque, un acide docosahexaenoïque et un acide linoléique.

**4.** Préparation nutritionnelle selon l'une quelconque des revendications précédentes dans laquelle la source protidique contient :
   - une protéine de haute valeur biologique
   - des acides aminés
   - des acides aminés à chaîne ramifiée et
   - de la L-carnitine.

**5.** Préparation nutritionnelle selon la renvendication 4 dans laquelle la protéine de haute valeur biologique est la lactalbumine ou la protéine de soja.

**6.** Préparation nutritionnelle selon les revendications 4 ou 5 dans laquelle les acides aminés contiennent : de la L-arginine; de la L-leucine; de la L-isoleucine; de la L-lysine; de la L-valine; de la L-phénylalanine; de la L-histidine; de la L-thréonine; de la L-méthionine; du L-tryptophane; de la L-alanine; de la L-proline; de la L-sérine; de la L-tyrosine; et un acide aminé acétique.

**7.** Préparation nutritionnelle selon les revendications 4 ou 5 dans lesquelles les acides aminés à chaîne ramifiée comprennent : de l'isoleucine, de la leucine et de la valine.

**8.** Préparation nutritionnelle selon l'une quelconque des revendications précédentes dans lesquelles la source d'hydrates de carbone contient des sous-unités de glucose et du xylitol.

**9.** Préparation nutritionnelle selon la revendication 8 dans laquelle la source de glucose est la maltodextrine.

**10.** Préparation nutritionnelle selon l'une quelconque des revendications précédentes dans lesquelles la source d'hydrates de carbone contient du ribose.

**11.** Préparation nutritionnelle selon l'une quelconque des revendications précédentes dans lesquelles l'acide gras à chaîne moyenne contient 50 à 75 % de la teneur totale en lipides de la préparation.

**12.** Préparation nutritionnelle selon l'une quelconque des revendications précédentes comportant une quantité thérapeutiquement efficace d'électrolytes.

**13.** Préparation selon l'une quelconque des revendications précédentes étant une préparation entérale dans laquelle la source protidique contient de 1a L-carnitine.

**14.** Préparation selon la revendication 3 ou l'une quelconque de ses revendications dépendantes, étant une préparation entérale et contenant de l'acide gammalinoléique et de l'acide stérodonique.

**15.** Régime parentéral pour traitement cardiaque comprenant :
- des quantités thérapeutiquement efficaces d'une émulsion lipidique contenant une huile de triacylglycérol ayant au moins un des lipides choisis dans le groupe contenant de l'acide eicosapentaenoïque, de l'acide gamma-linoléique, et de l'acide stérodonique, et un phospholipide sélectionné à partir d'un groupe consistant en phospholipide d'oeuf ou en phospholipide de soja, glycérol et eau, l'huile de triacylglycérol contenant 5 % à 20 % de l'émulsion lipidique
- une solution injectable d'un hydrate de carbone
- de la L-carnitine
- une solution injectable d'acides aminés ramifiés en chaîne et
- une solution injectable d'acides aminés

**16.** Régime parentéral selon la revendication 15 dans lequel l'huile de triacylglycérol contient 5 à 80 % d'acide eicosapentaenoïque et/ou 5 à 80 % d'acide gamma-linoléique et/ou 3 à 35 % d'acide stérodonique.

**17.** Régime parentéral selon la revendication 15 ou 16 dans lequel les acides aminés incluent : de la L-arginine; de la L-leucine; de la L-isoleucine; de la L-lysine; de la L-valine; de la L-phénylalanine; de la L-histidine; de la L-thréonine; de la L-méthionine; du L-tryptophane; de la L-alanine; de la L-proline; de la L-sérine; de la L-tyrosine; et un acide aminé acétique.

**18.** Régime parentéral selon la revendication 15 ou 16 dans lequel les acides aminés à chaîne ramifiée comprennent de l'isoleucine, de la leucine et de la valine..

**19.** Régime parentéral selon l'une quelconque des revendications de 15 à 18 dans lequel l'émulsion lipidique, la solution d'hydrates de carbone, la L-carnitine, les acides aminés à chaîne ramifiée, et les acides aminés sont pré-mélangés avant la perfusion au patient.

**20.** Régime parentéral selon l'une quelconque des revendications 15 à 19 dans lequel l'émulsion lipidique comprend des triglycérides à chaîne moyenne.

14

**21.** Régime parentéral selon l'une quelconque des revendications 15 à 20 dans lequel la solution d'hydrates de carbone contient du glucose et du xylitol.

**22.** Utilisation, dans la fabrication d'une préparation nutritionnelle entérale, de quantités thérapeutiquement efficaces d'une source protidique, d'une source d'hydrates de carbone et d'un composant lipidique comportant au moins un acide gras à chaîne moyenne et des triglycérides à longue chaîne comprenant de l'acide gamma-linoléique et/ou de l'acide stérodonique et/ou des huiles de poisson, en quantité comprenant 50 à 25 % du composant lipidique, indiquée dans la nutrition des invidus susceptibles de souffrir de maladies athéroscléreuses, vasculaires, cardiovasculaires et/ou thrombotiques ou en traitement pour ces maladies.

**23.** Utilisation, dans la fabrication d'un régime parentéral, de quantités thérapeutiquement efficaces d'une émulsion lipidique comprenant une huile de triacylglycérol ayant au moins l'un des lipides sélectionnés parmi l'acide eicosapentaenoïque, l'acide gamma-linoléique et l'acide stérodonique, et un phospholipide choisi à partir de phospholipide d'oeuf ou de soja, et du glycérol et de l'eau, l'huile de triacylglycérol comprenant 5 à 20 % de l'émulsion lipidique; une solution injectable d'un hydrate de carbonne; de la L-carnitine, une solution injectable d'acides aminés à chaîne ramifiée; et une solution injectable d'acides aminés, indiquée dans la nutrition des individus susceptibles de souffrir de maladies athéroscléreuses, vasculaires, cardiovasculaires et/ou thrombotiques ou en traitement pour ces maladies.